# EUROPEAN PATENT SPECIFICATION

(11) **EP 0 594 592 B1**
(45) Date of publication and mention of the grant of the patent: **23.04.1997**
(21) Application number: 91904533.6
(22) Date of filing: 04.02.1991
(51) Int. Cl.: A61K 38/06, A61L 27/00

(54) **A GROWTH FACTOR IN CONNECTION WITH ARTIFICIAL TRANSPLANTS**
WACHSTUMSFAKTOR IN VERBINDUNG MIT KÜNSTLICHEN TRANSPLANTATEN
FACTEUR DE CROISSANCE UTILISE EN RAPPORT AVEC DES TRANSPLANTS ARTIFICIELS

(30) Priority: 06.02.1990 SE 9000409
(43) Date of publication of application: 04.05.1994
(73) Proprietor: Strid, Lars, S-413 20 Göteborg (SE)
(72) Inventor: Strid, Lars, S-413 20 Göteborg (SE)
(74) Representative: Graudums, Valdis
(86) International application number: SE9100079
(87) International publication number: WO9112014

(56) References cited:
- EP-A- 0 190 736
- EP-A- 0 358 819
- WO-A-88/03417
- WO-A-88/08714
- WO-A-89/12441

## Description

### Technical field:

This invention utilizes the biological activity of the copper complex of the tripeptide glycyl-L-histidyl-L-lysine. This peptide is covalently bound to artificial implants where it has a chemoattractive effect and also acts when it is released by hydrolysis of the implants. The peptide increases fibroblastic collagen synthesis thereby enabling a more rapid replacement of the implants with human tissue.

### Background:

The evolution of multicellular organisms is dependent upon the ability of cells to communicate with each other and with their environment. One method the cells use for this communication is to release peptides that induce a specific activity in the receiving cell.

Such a peptide with biological activity is the human plasma growth factor copper-binding tripeptide glycyl-L-histidyl-L-lysine (GHK-Cu²⁺).

L Pickart and S Lovejoy describe the properties of the peptide GHK-Cu²⁺ in Methods in Enzymology, Vol 147 (1987) pp 314-328, Academic Press. It plays a physiological role in the healing of wounds by stimulation the complex course of events necessary for the formation of new tissues such as angiogenesis and axon and dendrite growth in neurons. The peptide has also a chemoattractive effect on cells necessary for wound-healing such as macrophages, monocytes, mast cells and capillary endothelial cells.

Collagen is a fibrous protein that constitutes a quarter of the total amount of protein in the human body. It is the major fibrous element of skin, bone, tendons,cartilage, ligaments and blood vessels. Collagen is synthesized by fibroblasts, a type of cell localized in the area surrounding other cells and tissues.

In a publication in FEBS Letters 238 (1988) 343-346, F-X Maquart et al present data showing that GHK-Cu²⁺ stimulates collagen synthesis in cultures of fibroblasts. This stimulation is observed at a peptide concentration as low as 10⁻¹²M and reaches a maximum at 10⁻⁹M where the increase in collagen synthesis is about 80 %.

In European Patent Appliction 0190736, GHK-Cu²⁺ with a modified C-terminal carboxyl group is used as an ointment for faster healing of wounds.

GHK-Cu²⁺ possesses a significant superoxide dismutase-like activity with a rate constant of about 25 % of the activity of enzymatic Cu,Zn-superoxide dismutase on a molar basis. When wounds and damaged tissue are present, cells from the immune system invade the injured area and large quantities of toxic oxygen radicals are released to kill invading bacteria. These radicals also destroy intact tissue which starts a vicious circle where more radicals are released, thus delaying healing. GHK-Cu²⁺ superoxide dismutase activity detoxifies the tissue destroying superoxide anions.

Aggregation of blood platelets is the first stage of thrombosis. GHK-Cu²⁺ inhibits this aggregation and it also inhibits the hormone thromboxane which causes thrombosis.

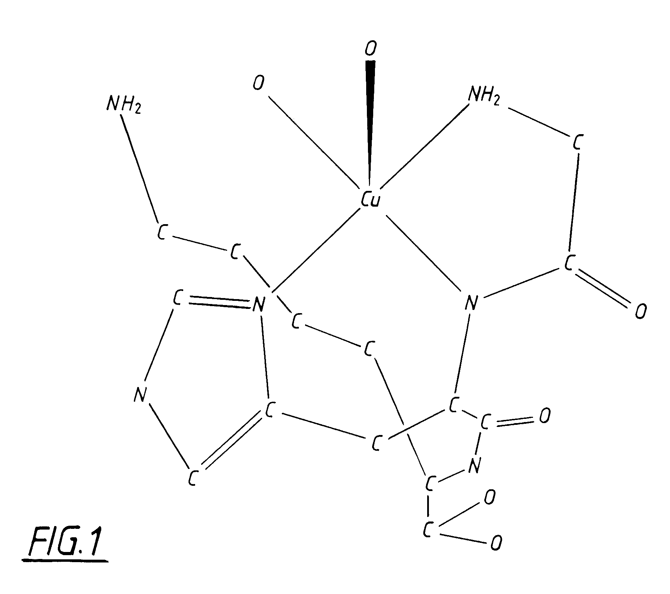

The structure of the tripeptide GHK-Cu²⁺ is shown in Fig 1. The affinity of the peptide for copper is very high with a pK for the dissociation constant of about 16. For biological activity the ε-amino group on the side-chain of the lysine must be free.

### The technical problem:

The use of artificial materials in surgery is gradually increasing. In orthopedic surgery, implants are used for soft parts such as muscles, tendons and ligaments. In the case of ligaments, the preferred material used has been polypropylene bands which are unfortunately not degradable, and results have been unfavorable in the long run. When degradable material is used to replace ligaments, it is of utmost importance that this material is biocompatible. Polymers of lactic acid and glycolic acid are degraded to non-toxic products that living tissues tolerate. In the human body they are hydrolysed to their monomers. Lactic acid is metabolized to carbondioxide and water in the citric acid cycle while glycolic acid is excreted with the urine or is oxidized to pyruvate and is also metabolized in the citric acid cycle resulting in the same products as for lactic acid. These harmless products are very advantageous when compared with the hydrolysis products of many other polymers.

Polymers of lactic and glycolic acid have been used for long time as resorbable suture material. When this material is used for artificial ligaments the main problem is that they hydrolyse so rapidly that the body is not allowed enough time to replace them with its own tissue where collagen constitutes the main part. The rapid hydrolisis could be compensated for if one could increase the rate of collagen synthesis.

### The solution:

The invention presented here is that material used for implants incorporates the copper-complex of the tripeptide glycyl-L-histidyl-L-lysine GHK-Cu²⁺. As may be seen in Fig 1, the C-terminal carboxyl group is not involved in the copper binding and for that reason is suitable for the covalent binding of the peptide to implants.

With well-known peptide synthesis, the free carboxyl group of the peptide is coupled to a polymer containing free primary amino groups or to a polymer which can be modified to contain free amino groups. Since the peptide has maximal activity at a concentration as low as 10⁻⁹M where it almost doubles the fibroblasts synthesis of collagen, it is fully sufficient that one peptide molecule is coupled to a polymer molecul having a molecul weight of 500 000.

Thought of chemoattraction and when the peptide GHK-Cu²⁺ is released by hydrolysis of the implant in the tissues, very specific biological processes are started involving many closely coordinated reactions that must be in balance in order to allow healing to occur. This includes an increase in collagen synthesis, an increase of tissue-protective superoxide dismutase activity, and a chemoattractive effect on among others mast cells and capillary endothelial cells which accumulate at the implant site, there stimulating new formation of blood vessels and the flow nourishment to the area.

### Best mode of carrying out the invention:

### Example 1.

The free carboxyl group of poly-L-lactic acid are activated with carbodiimide and then allowed to react with an excess of a diamine, NH₂(CH₂)ₙNH₂ where n = = 2-6 depending upon the length of the desired spacer-arm. The carbodiimide will be dicyclohexylcarbodiimid if the reaction is carried out in organic solution, or 1-ethyl-3-(3-dimethylaminopropyl-)carbodiimide if the reaction is performed in a water solution.

The tripeptide NH₂-glycyl-L-histidyl-L-lysine-COOH is blocked on the amino group of glycine, the imidazole group of histidine and the ε-amino group of lysine with 9-fluorenylmethyl chloroformate (FMOC-Cl). The free carboxyl group of lysine is activated with carboldiimide as described above and allowed to react with the free amino group of the derivative of poly-L-lactic acid [poly-L-lactic acid-C-NH(CH₂)ₙ-NH₂].

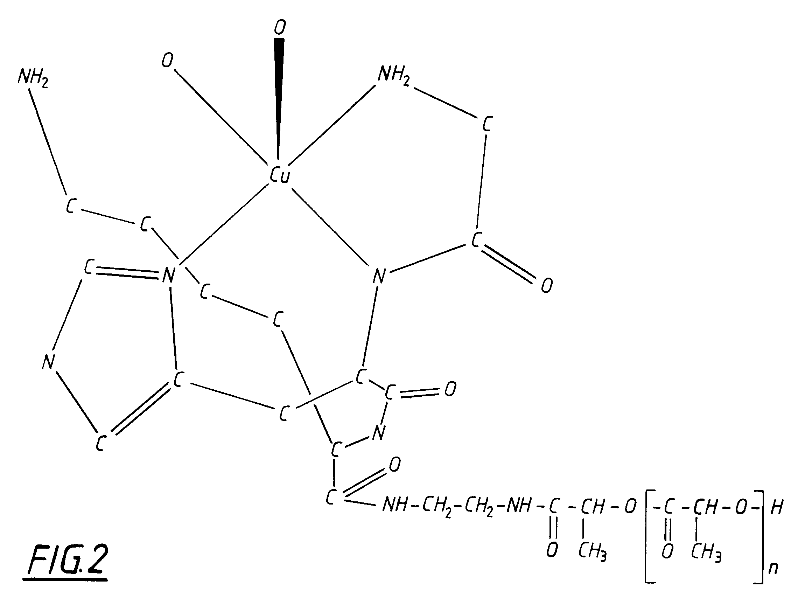

After removal of the protective FMOC-groups with piperidine and the addition of copper (II) acetate the desired product is obtained, the structure of which is shown in Fig 2. In this figure the diamine NH₂(CH₂)₂NH₂ has been used. The same methods of synthesis are also valid for poly-DL-lactic acid, poly-D-lactic acid, polyglycolic acid and their co-polymers.

Above, FMOC has been used as the blocking group. Another blocking agent is the tert-butoxycarbonyl group (t-BOC-). Deblocking after the synthesis is in this case performed with dilute acid (25% trifluoroacetic acid).

The peptide glycyl-L-histidyl-L-lysine is commercially available.

### Other examples

Another way for synthesis is by usual peptide synthesis coupling the amino blocked L-lysine, L-histidine and glycine one after the other to the amino derivative of the polymer.

Lactic acid and glycolic acid are α-hydroxycarboxylic acids. A number of other α-hydroxycarboxylic acids are useful for homo- or co-polymerization. Examples are α-hydroxybutyric acid, α-hydroxysiobutyric acid, α-hydroxyvaleric acid, α-hydroxyisovaleric acid. To increase the amount of peptides per polymer molecule, one can treat the poly-α-hydroxycarboxylic acid with mild hydrolysis to increase the available number of carboxyl groups.

Among β-hydroxycarboxylic acid D-β-hydroxybutyric acid has been used as a polymer for implants (British Patent 1034123).

Polymer based on p-dioxanon has also been used as implants. This has the formula 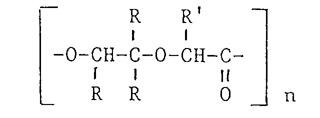 where R and R' represent hydrogen, methyl- or ethyl-group and n the degree of polymerization.

Synthetic biodegradable polyester amides have been described by T H Barrows et al at 3M Center, St Paul, Minnesota, USA.

These polymers consist of units with the formula

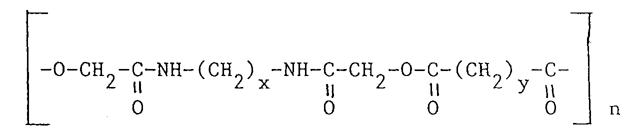

All of these polymers can be coupled covalently to GHK-Cu²⁺ with the same methods described above.

Another method is to covalently couple GHK-Cu²⁺ to other biocompatible materials, which may or may not be resorbed, that contain an amino group or a site where an amino group can be introduced. For example glass or silicic acid can be silylated with 3-aminopropyl-triethoxysilone. In this case a great number of GHK-Cu²⁺ could be bound to the glass.

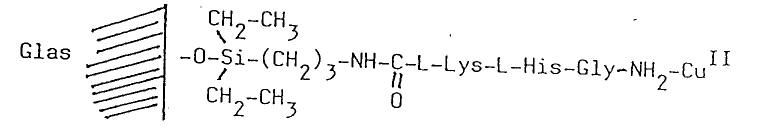

When metal implants are to be made the surface can first be covered with a protein, for instance serumalbumin, and the amino groups bridge-bound with glutardialdehyde. After introduction of new amino groups, the GHK-CU²⁺ can be bound to the protein.

Fig 1 shows the three-dimensional structure of GHK-Cu²⁺. The side-chains of lysine and histidine and the amino terminal of glycin are necessary for the copper-binding and for the biological activity. One of the hydrogens of the α-carbon in the glycin can be exchanged with another radical without disturbing the conformation of the copper-complex. Proposals for new tripeptides can, for example, be Ala-His-Lys, Val-His-Lys or Leu-His-Lys. One advantage of this is that we can use the D-form of the amino-terminal amino acid which renders the peptide more resistant to proteolytic hydrolysis.

In the practical use of the invention reinforcing material can of course be used. This material can be degradable or not degradable.

## Claims

1. Artificial implant having the ability of increasing natural collagen synthesis, **characterized** in that it contains the coppercomplex of the tripeptide glycyl-L-histidyl-L-lysine which is immobilized on the implant through a covalent bound of the carboxyl group of the lysine.

2. Implant according to claim 1,
**characterized** in that it consists of poly-L-lactic acid, polyglycolic acid or copolymer thereof, where the carboxyl group of the polymer has first been bound by a peptidebond to a diamine, preferably 1,6, -hexandiamine, and where the free amino group of the product is bound by a peptide bond to the carboxyl group of the lysine in the copper complex of the tripeptide glycyl-L-histidy-L-lysine (GHK-Cu²⁺).

3. Implant according to claim 2,
**characterized** in that the implants are homo- or copolymers of glycolic acid, lactic acid, the α-hydroxy-derivatives of butyric acid, isobutyric acid, valeric acid and isovaleric acid.

4. Implant according to claim 2,
**characterized** in that the implant is a polymer based on p-dioxanone consisting of units with the formula 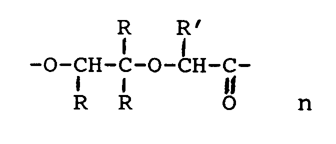 where R and R' indicate hydrogen, methyl or ethyl group and n the degree of polymerization.

## Patentansprüche

1. Künstliches Implantat mit der Fähigkeit, die natürliche Collagensynthese zu erhöhen,
**dadurch gekennzeichnet**, daß
es den Kupferkomplex des Tripeptids Glycyl-L-Hystidyl-L-Lysin enthält, der auf dem Implantat durch eine kovalente Bindung der Carboxylgruppe des Lysins immobilisiert ist.

2. Implantat nach Anspruch 1,
**dadurch gekennzeichnet**, daß
es aus Poly-L-Milchsäure, Polyglycolsäure oder einem Copolymer hiervon besteht, wobei die Carboxylgruppe des Polymers zunächst durch eine Peptidbindung an ein Diamin, bevorzugt 1,6-Hexandiamin, gebunden wurde, und wobei die freie Aminogruppe des Produkts durch eine Peptidbindung an die Carboxylgruppe des Lysins im Kupferkomplex des Tripeptids Glycyl-L-Hystidyl-L-Lysin (GHK-Cu²⁺) gebunden wurde.

3. Implantat nach Anspruch 2,
**dadurch gekennzeichnet**, daß
die Implantate Homo- oder Copolymere der Glycolsäure, Milchsäure, der α-Hydroxy-Derivate der Buttersäure, Isobuttersäure, Valeriansäure und der Isovaleriansäure sind.

4. Implantat nach Anspruch 2,
**dadurch gekennzeichnet**,
daß das Implantat ein auf p-Dioxanon basierendes Polymer ist, bestehend aus Einheiten mit der Formel 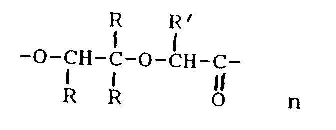 wobei R und R' eine Wasserstoff-, Methyl- oder Ethylgruppe sind und n der Polymerisationsgrad ist.

## Revendications

1. Implant artificiel ayant la capacité d'accroître la synthèse naturelle du collagène, caractérisé en ce qu'il contient le complexe avec le cuivre du tripeptide glycyl-L-histidyl-L-lysine immobilisé sur l'implant par liaison covalente du groupe carboxyle de la lysine.

2. Implant selon la revendication 1, caractérisé en ce qu'il est constitué de poly(acide L-lactique), de poly(acide glycolique) ou d'un copolymère de ces derniers où le groupe carboxyle du polymère a d'abord été lié par une liaison peptide à une diamine, de préférence 1,6, -hexanediamine, et où le groupe amine libre du produit est lié par une liaison peptide au groupe carboxyle de la lysine dans le complexe avec le cuivre du tripeptide glycyl-L-histidyl-L-lysine (GHK-Cu²⁺).

3. Implant selon la revendication 2, caractérisé en ce que les implants sont des homopolymères ou des copolymères de l'acide glycolique, de l'acide lactique, les dérivés α-hydroxy de l'acide butyrique, de l'acide isobutyrique, de l'acide valérique et de l'acide isovalérique.

4. Implant selon la revendication 2, caractérisé en ce que l'implant est un polymère à base de p-dioxanone constitué de motifs de formule 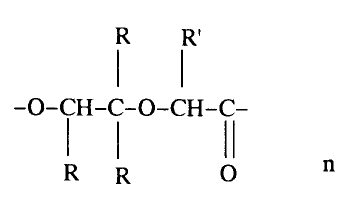 où R et R' désignent un atome d'hydrogène, un groupe méthyle ou éthyle et n le degré de polymérisation.
